Europäisches Patentamt

European Patent Office

Office européen des brevets

(11) Veröffentlichungsnummer: **0 365 705**
**A1**

## (12) EUROPÄISCHE PATENTANMELDUNG

(21) Anmeldenummer: 88117869.3

(22) Anmeldetag: 26.10.88

(51) Int. Cl.⁵: **A61K 37/12 , A61K 37/547 ,**
**//(A61K37/12,31:47,31:11,**
**31:195),(A61K37/547,37:12,**
**31:47,31:11)**

(43) Veröffentlichungstag der Anmeldung:
**02.05.90 Patentblatt 90/18**

(84) Benannte Vertragsstaaten:
**AT CH DE FR GB GR LI**

(71) Anmelder: **ZENTRALNA PROBLEMNA LABORATORIA PO KRYOBIOLOGIA I LYOPHILISAZIA**
**Boul. Tcherni Vrah Nr. 65**
**BG-1407 Sofia(BG)**

(72) Erfinder: **Tzvetkov, Tzvetan Dimitrov, Dipl.-Ing.**
**Iliya Filipov Strasse 54-B**
**Sofia(BG)**
Erfinder: **Metchkarski, Stefan Ninov, Dr.**
**Viktor Grigorovitch-Strasse 12**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Biopräparat zur Behandlung von Wunden.**

(57) Das Biopräparat zur Behandlung von Wunden verschiedener Ursache enthält eine Kollagenlösung oder eine Lösung seines Hydrolyseproduktes, 5-Nitro-8-hydroxychinolin, Glutaraldehyd und als aktiv wirkede Substanz Thrombin oder Epsilon-Aminocapronsäure in folgendem Mengenverhältnis in Gewichtsprozent:

| | | | |
|---|---|---|---|
| Kollagenlösung oder Lösung seines Hydrolyseproduktes | 96,0 | bis | 99,0% |
| 5-Nitro-8-hydroxychinolin | 0,05 | bis | 1,5% |
| Glutaraldehyd | 0,10 | bis | 0,5% |
| aktiv wirkende Substanz | 0,05 | bis | 2,0%. |

Statt Kollagenlösung kann auch Gelatinelösung bei der Herstellung des Biopräparates vorliegen.
Das Biopräparat weist bei seiner Anwendung in der Humanmedizin eine stark ausgeprägte hämostatische Wirkung auf.

## BIOPRÄPARAT ZUR BEHANDLUNG VON WUNDEN

Die Erfindung betrifft ein Biopräparat zur Behandlung von Wunden verschiedener Ursachen, wie Verbrennungen, Schnittwunden und Wunden von Feuerwaffen usw., das eine stark ausgeprägte hämostatische Wirkung aufweist und in der Humanmedizin angewendet wird.

Es ist bekannt, Kollagenschwämme, die vor ihrer Verwendung in eine Thrombinlösung getaucht wurden, auf die Wunde aufzutragen. Als Nachteil dieses Schwammes gilt, daß er seine Konsistenz ändert, keine schnelle und dauerhafte Hämostase hervorruft, keine tamponierende Wirkung aufweist und seine Adhäsion zur Wunde zu schwach ist. Solche Schwämme werden nur bei oberflächlichen Verletzungen der Parenchymatosenorgane angewendet. Sie haben keine antiseptische Wirkung (V.V. Kovanov, I.A. Sitschenikov, "Kollagenplastik in der Medizin", 1978, Seiten 58-62).

Es ist ein Präparat auf Gelatinebasis bekannt, das als hämostatisches Mittel bei blutenden Wunden angewendet wird (Stein Jand, Hornick A., "Biobran as Dressing for Excised Burn Wounds", Sixth Suter Congress on Burns, September 1982).

Der Erfindung liegt die Aufgabe zugrunde, ein Biopräparat zur Behandlung von Wunden auf der Basis von Kollagen oder seinem Hydrolyseprodukt zu entwickeln, das eine schnelle und dauerhafte Hämostase hervorruft, eine gut ausgeprägte tamponierende Wirkung aufweist, eine große Adhäsionsfähigkeit besitzt, eine unveränderliche Konsistenz während seiner Anwendung aufweist, die Möglichkeit zur Anwendung bei operativen Wunden der Parenchymatosenorgane bietet, einen guten Schutz gegen bakterielle Außeninvasionen sichert, eine epithelisierende Wirkung besitzt und dazu noch steril ist.

Diese Aufgabe wird durch die Entwicklung eines Biopräparates zur Behandlung von Wunden gelöst, das eine gut ausgeprägte hämostatische Wirkung aufweist und folgende Zusammensetzung aufweist (in Gewichts-%):

| | |
|---|---|
| Kollagenlösung oder Lösung seines Hydrolyseproduktes | 96,0 bis 99,0% |
| 5-Nitro-8-hydroxychinolin | 0,05 bis 1,5% |
| Glutaraldehyd | 0,1 bis 0,5% |
| aktiv wirkende Substanz | 0,05 bis 2,0%. |

Das erfindungsgemäße Biopräparat enthält als aktiven Stoff Thrombin oder Epsilon-Aminocapronsäure und kann auf ein Polyamidsieb oder ein anderes Sieb aufgetragen werden. Das Präparat wird in vier Varianten, in Abhängigkeit der pharmakologischen Orientierung des aktiv wirkenden Stoffes und der Verwendung der Kollagenlösung oder der Lösung seines Hydrolyseproduktes, vorgeschlagen.

| | | | |
|---|---|---|---|
| Kollagenlösung | 96,0 | bis | 99,0% |
| 5-Nitro-8-hydroxychinolin | 0,05 | bis | 1,5% |
| Glutaraldehyd | 0,10 | bis | 0,5% |
| Thrombin | 0,05 | bis | 2,0%; |
| Kollagenlösung | 96,0 | bis | 99,0% |
| 5-Nitro-8-hydroxychinolin | 0,05 | bis | 1,5% |
| Glutaraldehyd | 0,10 | bis | 0,5% |
| Epsilon-Aminocapronsäure | 0,05 | bis | 2,0%; |
| Gelatinelösung | 96,0 | bis | 99,0% |
| 5-Nitro-8-hydroxychinolin | 0,05 | bis | 1,5% |
| Glutaraldehyd | 0,10 | bis | 0,5% |
| Thrombin | 0,05 | bis | 2,0%; |
| Gelatinelösung | 96,0 | bis | 99,0% |
| 5-Nitro-8-hydroxychinolin | 0,05 | bis | 1,5% |
| Glutaraldehyd | 0,10 | bis | 0,5% |
| Epsilon-Aminocapronsäure | 0,05 | bis | 2,0%. |

Das Biopräparat wird durch Mischen der einzelnen Bestandteile hergestellt. Die verwendete Kollagenlösung wurde durch Enzymbehandlung mit sauren Proteasen erhalten. Zu der Kollagenlösung oder zur Lösung seines Hydrolyseproduktes werden unter kontinuierlicher Homogenisierung nacheinander die einzelnen Komponenten zugefügt. Die homogene Mischung wird dann einer Reihe von technologischen Operationen, die eine stabile und sterile therapeutische Endform sichern, unterzogen.

Das Biopräparat enthält erfindungsgemäß Thrombin, wenn es bei Blutungen der Parenchymatosenorgane (Leber, Milz, Nieren) wegen Traumas oder chirurgischen Eingriffen oder als Ergebnis von Cholezystektomie, Prostatektomie, Anastomose der Blutgefäße, Füllung der Geschwulstorte nach ihrer Beseitigung, bei Blutungen spongiöser Knochen, Tamponade, Epistaxis, Tonselektomie usw. verwendet werden, wobei das durchgerissene Organ genäht wird oder ohne Naht bleibt.

Das Biopräparat, das erfindungsgemäß Epsilon-Aminocapronsäure enthält, wird bei verschiedenen Arten von Wunden, wie Verbrennungen,Trauma, Ostheomyelit, atrophische Wunden usw., eingesetzt.

Der Vorteil des Biopräparates besteht darin, daß es eine gut ausgeprägte, schnelle und dauerhafte hämostatische Wirkung, einen tamponierenden Effekt und eine gute Adhäsionsfähigkeit aufweist und weiterhin seine Konsistenz während der Anwendung nicht ändert, eine epitelisierende Wirkung zeigt und vollkommen steril ist.

Beispiel 1

| Kollagenlösung (4%ig) | 98,0% |
| 5-Nitro-8-hydroxychinolin | 1,0% |
| Glutaraldehyd (25%) | 0,5% |
| Thrombin | 0,5% |

Das Biopräparat wird bereitet, indem man zur Kollagenlösung bei kontinuierlicher Homogenisierung die einzelnen Bestandteile zufügt. Das so erhaltene homogene Gemisch wird in Gefäßen zur Lyophilisierung dosiert. Das Produkt wird 24 h bei -4°C eingefroren. Danach wird es lyophilisiert, gewogen, abgepackt und gamma-sterilisiert.

Beipsiel 2

| Kollagenlösung (4%ig) | 96,0% |
| 5-Nitro-8-hydroxychinolin | 1,5% |
| Glutaraldehyd (25%) | 0,5% |
| Epsilon-Aminocapronsäure | 2,0% |

Das Biopräparat wird gemäß Beispiel 1 hergestellt.

Beipiel 3

| Gelatinelösung (4%ig) | 99,0% |
| 5-Nitro-8-hydroxychinolin | 0,75% |
| Glutaraldehyd (25%) | 0,2% |
| Thrombin | 0,05% |

Das Biopräparat wird gemäß Beispiel 1 hergestellt.

Beispiel 4

| Gelatinelösung (4%ig) | 97,0% |
|---|---|
| 5-Nitro-8-hydroxychinolin | 1,0% |
| Glutaraldehyd (25%) | 0,2% |
| Epsilon-Aminocapronsäure | 1,8% |

Das Biopräparat wird gemäß Beispiel 1 hergestellt.

Beispiel 5

Das Biopräparat mit Gehalt an Thrombin wurde an 24 Kaninchen und 20 Hunden mit Abrißquetschungswunden der Leber und Milz erprobt. Das Präparat ruft eine schnelle lokale Hämostase hervor, wobei kein Koagulum zwischen dem Präparat und der blutenden Oberfläche gebildet wird; es verliert sein Volumen und seine Ganzheit nach dem Vollsaugen mit Blut nicht; es bewahrt seine Adhäsionseigenschaften und verhindert die Entwicklung lokaler Infektionen bakteriellen Ursprungs. Während des Experiments wurden die hämostatische Wirkung (erforderliche Zeit zur Bildung der lokalen Hämostase), die Adhäsion zur Wundoberfläche, der mechanische Effekt bei Anwendung des Präparates und das Auftragen auf eine blutende Oberfläche, die Toleranz, die Resorption, die Zeitdauer zur Heilung einer operativen Wunde und die Bestimmung von Indikationen zur klinischen Anwendung des Präparates untersucht.

Das Biopräparat wurde noch an 25 Kaninchen bei eindringendem Schädel-Hirn-Trauma erprobt. Bei allen untersuchten Tieren wurde eine vollkommene Hämostase bei Gehirnblutungen nach Anwendung des Präparates festgestellt. Es wird gut vom Organismus vertragen, es wird nicht abgestoßen und übt keine Nebenwirkungen aus.

Beispiel 6

Es wurden hystomorphologische Untersuchungen an 10 Hunden der Rasse Chinchilla durchgeführt.

Es wurde eine Abrißquetschungswunde am konvexen Überzug der Leber und der Milz verursacht. Die Wunde wurde mit dem Biopräparat mit Thrombingehalt überzogen. Es wurde festgestellt, daß es gut vom Organismus vertragen wird, nicht abgestoßen wird, es verursacht ferner keine Reaktionen des Typs "Fremdkörper" und es wurden keine lokalen toxischen und allergischen Wirkungen festgestellt.

Beispiel 7

Es wurden klinische Untersuchungen des erfindungsgemäßen Präparates nach Beispiel 1 und Beispiel 3 durchgeführt, indem man es bei verschiedenen Erkrankungen, wie Trauma der Leber und der Milz, Cholezystektomie usw., eingesetzt hat. Das Biopräparat unterbricht die Blutungen nach 30 s bis 1 min. Es verursacht keine Nebenwirkungen und wird vom Organismus sehr gut vertragen.

Beispiel 8

Das Biopräparat gemäß Beispiel 2 und Beispiel 4 wurde bei 30 Patienten mit Oberflächenverbrennungen II. und III. Grades erprobt. Die Wirkung das Präparates führt zur beschleunigten Epitelisierung und verkürzt die Zeitdauer der Vorbereitung für operative Behandlungen.

**Ansprüche**

1. Biopräparat zur Behandlung von Wunden auf der Basis von Kollagen oder seines Hydrolyseproduktes, **gekennzeichnet dadurch,** daß es zusätzlich 5-Nitro-8-hydroxychinolin, Glutaraldehyd und eine aktiv

4

EP 0 365 705 A1

wirkende Substanz mit orientierter Wirkung in folgendem Mengenverhältnis in Gewichts-% enthält:

| | | | |
|---|---|---|---|
| Kollagenlösung oder Lösung seines Hydrolyseproduktes | 96,0 | bis | 99,0% |
| 5-Nitro-8-hydroxychinolin | 0,05 | bis | 1,5% |
| Glutaraldehyd | 0,10 | bis | 0,5% |
| aktiv wirkende Substanz | 0,05 | bis | 2,0%. |

2. Biopräparat gemäß Anspruch 1, **gekennzeichnet dadurch,** daß es Kollagen enthält und als aktiv wirkende Substanz Thrombin.

3. Biopräparat gemäß Anspruch 1, **gekennzeichnet dadurch,** daß es Kollagen und als aktiv wirkende Substanz Epsilon-Aminocapronsäure enthält.

4. Biopräparat gemäß Anspruch 1, **gekennzeichnet dadurch,** daß es Gelatinelösung und als aktiv wirkende Substanz Thrombin enthält.

5. Biopräparat gemäß Anspruch 1, **gekennzeichnet dadurch,** daß es Gelatinelösung und als aktiv wirkende Substanz Epsilon-Aminocapronsäure enthält.

5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 637 815 (G.M. LEMOLE)<br>* Anspruch 1 *<br>--- | 1-3 | A 61 K 37/12<br>A 61 K 37/547//<br>(A 61 K 37/12 |
| A | US-A-4 006 220 (S.K. GOTTLIEB)<br>* Ansprüche 1-3 *<br>--- | 1,5 | A 61 K 31:47<br>A 61 K 31:11<br>A 61 K 31:195)<br>(A 61 K 37/547 |
| A | DE-A-3 146 841 (BEIERSDORF)<br>* Ansprüche 1,2 *<br>----- | 1 | A 61 K 37:12<br>A 61 K 31:47<br>A 61 K 31:11 ) |

RECHERCHIERTE SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 29-06-1989 | PEETERS J.C. |